(19) Europäisches Patentamt
European Patent Office
Office européen des brevets

(11) Publication number: **0 551 617 A2**

# (12) EUROPEAN PATENT APPLICATION

(21) Application number: **92121119.9**

(22) Date of filing: **11.12.92**

(51) Int. Cl.5: **C07D 295/06**, C07D 295/02, C07D 295/08, C07D 317/58, A01N 43/60

(30) Priority: **13.12.91 JP 360890/91**

(43) Date of publication of application: **21.07.93 Bulletin 93/29**

(84) Designated Contracting States: **DE ES FR GB IT**

(71) Applicant: **UBE INDUSTRIES, LTD.**
**12-32, Nishihonmachi 1-chome**
**Ube-shi, Yamaguchi(JP)**

(72) Inventor: **Nishimura, Takashi, c/o Ube Laboratory**
**Ube Industries, Ltd., 1978-5, Oaza Kogushi**
**Ube-shi, Yamaguchi(JP)**
Inventor: **Tanaka, Toshinobu, c/o Ube Laboratory**
**Ube Industries, Ltd., 1978-5, Oaza Kogushi**
**Ube-shi, Yamaguchi(JP)**
Inventor: **Takata, Katsunori, c/o Ube Laboratory**
**Ube Industries, Ltd., 1978-5, Oaza Kogushi**
**Ube-shi, Yamaguchi(JP)**
Inventor: **Moritomo, Masaru, c/o Ube Laboratory**
**Ube Industries, Ltd., 1978-5, Oaza Kogushi**
**Ube-shi, Yamaguchi(JP)**

(74) Representative: **Dr. Fuchs, Dr. Luderschmidt Dr. Mehler, Dipl.-Ing. Weiss Patentanwälte**
**Abraham-Lincoln-Strasse 7, Postfach 4660**
**W-6200 Wiesbaden (DE)**

## (54) Cinnamyl piperazine derivatives method of manufacturing the same and agricultural/horticultural fungicides containing cinnamyl piperazine derivatives.

(57) A new cinnamyl piperazine derivative represented by formula (I):

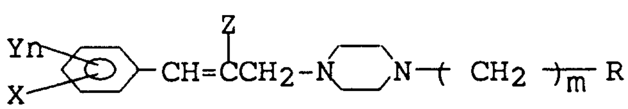

...(I)

(definitions of X, Y, Z, R, n and m are the same as shown in claim 1) and an acid-addition salt thereof, as well as the manufacturing method of the cinnamyl piperazine derivative and the acid-addition salt thereof are disclosed. These cinnamyl piperazine derivative and acid-addition salt thereof have distinguished sterilization activities as an agricultural/horticultural fungicide.

EP 0 551 617 A2

The present invention relates to new cinnamyl piperazine derivatives and acid-addition salts thereof, and to agricultural/horticultural fungicides containing the cinnamyl piperazine derivatives and/or their acid-addition salts as active ingredients.

Many conventional agricultural/horticultural fungicides such as triforine have been developed and conventionally used. However, sterilization activities of most of these fungicides are gradually weakened in long-term use as fungus resistances increase. Therefore, strong demand has arisen for a new and improved agricultural/horticultural fungicide having a stronger sterilization activity.

It is, therefore, an object of the present invention to provide a new and improved agricultural/horticultural fungicide having a strong sterilization activity.

The present inventors made extensive studies to solve the above problem, found that new cinnamyl piperazine derivatives had distinguished sterilization activities as the agricultural/horticultural fungicides, thereby achieving the present invention.

More specifically, according to the first aspect of the present invention, there is provided a cinnamyl piperazine derivative or an acid-addition salt thereof, the cynnamyl piperazine derivative being represented by formula (I):

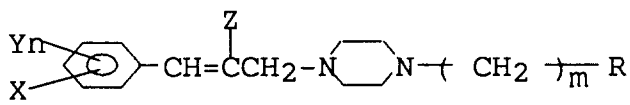

...(I)

(wherein each of X and Y independently represents: a hydrogen atom; a halogen atom; an alkyl group having 1 to 6 carbon atoms; a haloalkyl group having 1 to 4 carbon atoms; an alkoxy group having 1 to 4 carbon atoms, which may accompany a carbonyl group having an alkoxy group having 1 to 4 carbon atoms, a benzyloxycarbonyl group, a carbonyl group having an alkynyloxy group having 2 to 5 carbon atoms, or a cycloalkylalkyleneoxycarbonyl group having both cycloalkyl having 3 to 10 carbon atoms and alkylene having 1 to 8 carbon atoms; a nitro group; an alkoxycarbonyl group having 2 to 5 carbon atoms; a benzyloxy group; a hydroxyl group; or an alkylsulfonyloxy group having 1 to 4 carbon atoms,

X and Y may be condensed together with carbon atoms to which X and Y are bonded into a benzene ring, thereby forming a 5- or 6-membered ring containing two oxygen atoms,

Z represents an alkyl group having 1 to 10 carbon atoms, a phenyl group, or a halogen atom,

R represents a phenyl group which may have a substituent group, a cycloalkyl group having 3 to 10 carbon atoms, a phenoxy group which may have a substituent group, a benzyloxy group, a halogen atom, an alkyl group having 1 to 6 carbon atoms, an alkenyl group having 2 to 4 carbon atoms, or a phenylthio group,

m represents an integer of 1 to 10, and

n represents 1, 2, or 3).

Compound (I) of the present invention can be either an E isomer (trans isomer) or a Z isomer (cis isomer) as geometric isomers, and therefore it should be understood the definition of compound (I) includes a pure isomer and a mixture of isomers.

According to the second aspect of the present invention, there is provided a method of manufacturing a cinnamyl piperazine derivative represented by formula (I), wherein a compound represented by formula (II):

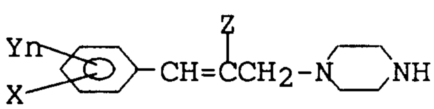

...(II)

(wherein X, Y, Z, and n are defined as described above) is reacted with a compound represented by formula (III):

$$W-(CH_2)_m-R \quad ...(III)$$

(wherein R and m are defined as described above, and W represents a leaving group).

According to the third aspect of the present invention, there is provided an agricultural/horticultural fungicide containing the cinnamyl piperazine derivative represented by formula (I) or its acid-addition salt as an active ingredient.

The constituents, X, Y, Z, m, n, and W of cinnamyl piperazine derivative (I) which is the target compound of the present invention and starting materials (II) and (III) for manufacturing the compound (I) will be described in detail below.

As described above, the definitions of X and Y includes:

a hydrogen atom;

a halogen atom;

a straight-chain or branched alkyl group having 1 to 6 carbon atoms;

a straight-chain or branched haloalkyl group having 1 to 4 carbon atoms;

a straight-chain or branched alkoxy group having 1 to 4 carbon atoms, which may accompany a carbonyl group having a straight-chain or branched alkoxy group having 1 to 4 carbon atoms, a carbonyl group having a straight-chain or branched alkynyloxy group having 2 to 5 carbon atoms, or a cycloalkylalkyleneoxycarbonyl group having both cycloalkyl having 3 to 10 carbon atoms and alkylene having 1 to 8 carbon atoms;

a nitro group;

a straight-chain or branched alkoxycarbonyl group having 2 to 5 carbon atoms;

a benzyloxy group;

a hydroxyl group; and

a straight-chain or branched alkylsulfonyloxy group having 1 to 4 carbon atoms.

Preferable examples of X and Y are as follows.

Preferable examples of the halogen atom are a chlorine atom, a bromine atom, and a fluorine atom.

Preferable examples of the straight-chain or branched alkyl group having 1 to 6 carbon atoms are alkyl groups having 1 to 4 carbon atoms (e.g., a methyl group, an ethyl group, an n-propyl group, an i-propyl group, an n-butyl group, an i-butyl group, and a t-butyl group), and more preferably a methyl group, an ethyl group, and an i-propyl group.

A preferable example of the straight-chain or branched haloalkyl group (e.g., a trifluoromethyl group, a difluoromethyl group, a 2-fluoroethyl group, a 2-chloroethyl group, a 2,2,2-trifluoroethyl group, and a 3,3,3-trifluoropropyl group) is a trifluoromethyl group.

Preferable examples of the straight-chain or branched alkoxy group (e.g., a methoxy group, an ethoxy group, an n-propoxy group, an i-propoxy group, an n-butoxy group, an i-butoxy group, and a t-butoxy group) are a methoxy group and an ethoxy group.

Examples of the straight-chain or branched alkynyloxy group having 2 to 5 carbon atoms are straight-chain or branched alkynyloxy groups having 3 to 4 carbon atoms (e.g., a 1-propynyloxy group, a 2-propynyloxy group, and a 2-butynyloxy group), and more preferably a 2-propynyloxy group.

Examples of the cycloalkyl group having 3 to 10 carbon atoms are preferably cycloalkyl groups having 5 to 8 carbon atoms, and more preferably a cyclohexyl group.

Examples of the alkylene group having 1 to 8 carbon atoms are preferably alkylene groups having 1 to 4 carbon atoms (e.g., methylene, ethylene, propylene, and butylene), and more preferably an ethylene group.

The most preferable example of the straight-chain or branched alkoxy group having 1 to 4 carbon atoms, which accompanies a carbonyl group having a straight-chain or branched alkoxy group having 1 to 4 carbon atoms is $-OCH_2COOC_2H_5$. The most preferable example of the straight-chain or branched alkoxy group having 1 to 4 carbon atoms, which accompanies a benzyloxycarbonyl group is $-OCH_2COOCH_2C_6H_5$. The most preferable example of the straight-chain or branched alkoxy group having 1 to 4 carbon atoms, which accompanies a carbonyl group having a straight-chain or branched alkinyloxy group having 2 to 5 carbon atoms is a propalgiloxycarbonylmethoxy group. The most preferable example of the straight-chain or branched alkoxy group having 1 to 4 carbon atoms, which accompanies a cycloalkylalkyleneoxycarbonyl group having both cycloalkyl having 3 to 10 carbon atoms and alkylene having 1 to 8 carbon atoms is $-OCH_2COOCH_2CH_2C_6H_{11}$. The substitution position of these alkoxy groups is not limited to a specific position, but is preferably the 2-position.

Examples of the straight-chain or branched alkoxycarbonyl group having 2 to 5 carbon atoms are straight-chain or branched alkoxy groups having 1 to 3 carbon atoms (e.g., a methoxy group, an ethoxy group, an n-propoxy group, and an i-propoxy group), and more preferably an ethoxycarbonyl group. The substitution position of these alkoxy groups is not limited to a specific position, but is preferably the 2-position.

Examples of the straight-chain or branched alkylsulfonyloxy group having 1 to 4 carbon atoms are sulfonyloxy groups having straight-chain or branched alkoxy groups having 1 to 3 carbon atoms (for example, the same materials as described above), and more preferably a methylsulfonyloxy group. The substitution position of these alkoxy groups is not limited to a specific position, but is preferably the 2-position.

Combinations of X and Y are as follows.

When X is a hydrogen atom and n in Y is 1, 2, or 3

Examples of Y include all those enumerated for Y above.

When X is a group other than the hydrogen atom and n in Y is 1 or 2

Examples of X and Y include all those enumerated for X and Y above. If n is 1, then preferable examples of Y are a hydrogen atom, a halogen atom, an alkoxy group having 1 to 4 carbon atoms (if X is a nitro group), a nitro group (if X is an alkoxy group having 1 to 4 carbon atoms), and an alkyl group having 1 to 4 carbon atoms. If n is 2, then preferable examples of Y are a hydrogen atom, a halogen atom, and an alkoxy group having 1 to 4 carbon atoms.

X and Y can be condensed together with carbon atoms to which X and Y are bonded into a benzene ring, thereby forming a 5- or 6-membered ring containing two oxygen atoms. A preferable example is a 5-membered ring (e.g., 3,4-($OCH_2O$)).

The positions of the substituent groups of X and Y are not limited to specific positions, but preferable positions are as follows. In the case of halogen atom, the positions of chlorine atom are preferably the 2-, 3-, 4-, 5-, and/or 6-position; the positions of fluorine atom are preferably the 2-, 3-, and/or 5-position; and the position of bromine atom is preferably the 2-position.

The positions of the substituent groups of the straight-chain or branched alkyl group having 1 to 6 carbon atoms are preferably the 2-, 4-, and/or 5-position. More preferably, the positions of the substituent groups of the methyl and ethyl groups are the 2-, 4-, and/or 5-position, and the position of the substituent group of the i-propyl group is preferably the 2- and/or 4-position.

In the case of: the straight-chain or branched haloalkyl group having 1 to 4 carbon atoms; the alkoxy group having 1 to 4 carbon atoms, which may accompany a carbonyl group having an alkoxy group having 1 to 4 carbon atoms, the benzyloxycarbonyl group, the carbonyl group having the alkynyloxy group having 2 to 5 carbon atoms, or the cycloalkylalkyleneoxycarbonyl group having both cycloalkyl having 3 to 10 carbon atoms and alkylene having 1 to 8 carbon atoms; the alkoxycarbonyl group having 2 to 5 carbon atoms; the benzyloxy group; the hydroxyl group; and the alkylsulfonyloxy group having 1 to 4 carbon atoms, the preferable position of the substituent group is the 2-position.

In the case of the nitro group, the preferable position of the subsitutent group is the 5-position.

In the case of the 5- or 6-membered ring containing two oxygen atoms which has been formed by the condensation of X and Y together with the carbon atoms to which X and Y are bonded into a benzene ring, the bonding positions of X and Y to the benzene ring are preferably the 3- and 4-positions.

m represents an integer of 1 to 10, preferably 2 to 8, and more preferably 2 to 7.

Examples of Z are an alkyl group having 1 to 10 carbon atoms, a phenyl group, and a halogen atom. An example of the alkyl group is preferably a straight-chain or branched alkyl group having 1 to 8 carbon atoms, and more preferably a straight-chain or branched alkyl group having 1 to 7 carbon atoms (for example, those listed as examples of X and Y, and n-$C_7H_{15}$). Preferable examples of the halogen atom are a bromine atom and a chlorine atom.

Examples of R are a phenyl group which may have a substituent group, a cycloalkyl group having 3 to 10 carbon atoms, a phenoxy group which may have a substituent group, a benzyloxy group, a halogen atom, an alkyl group having 1 to 6 carbon atoms, an alkenyl group having 2 to 4 carbon atoms, and a phenylthio group.

Preferable examples of R are as follows.

Preferable examples of the phenyl group which may have a substituent group are a phenyl group and a phenyl group having a substituent group.

Preferable examples of the substituent group in this phenyl group are: a straight-chain or branched alkoxy group having 1 to 4 carbon atoms (examples listed in the case of X and Y; among them a methoxy group is preferable, and the position of this substituent group is not limited to a specific position but is preferably the 3-position or 4-position); a phenoxy group (the position of this substituent group is not limited to a specific position, but is preferably the 4-position); a halogen atom (the materials enumerated for X and

Y are useful, but chlorine and fluorine atoms are preferable; and the position of this substituent group is not limited to a specific position but is preferably the 3- or 4-position for the chlorine atom and 2-, 3-, or 4-position for the fluorine atom); a straight-chain or branched alkyl group having 1 to 4 carbon atoms (the materials enumerated for X and Y are useful, but methyl and ethyl groups are preferable; and the position of this substituent group is not limited to a specific position, but is preferably the 2- or 4-position for the methyl group and the 2-position for the ethyl group); and a straight-chain or branched haloalkyl group having 1 to 4 carbon atoms (the materials enumerated for X and Y are useful, but a trifluoromethyl group is preferable; and the position of this substituent group is not limited to a specific position, but is preferably the 3-position).

An example of the cycloalkyl group having 3 to 10 carbon atoms is preferably a cycloalkyl group having 3 to 8 carbon atoms, more preferably a cycloalkyl group having 4 to 7 carbon atoms, and most preferably cyclopentane or cyclohexane.

Examples of the phenoxy group which may have the substituent group are a phenoxy group and a phenoxy group having a substituent group.

Examples of the substituent group in this phenoxy group are: a halogen atom (the materials enumerated for X and Y are useful, but a chlorine or fluorine atom is preferable; and the position of this substituent group is not limited to a specific position but is the 2-, 3-, or 4-position for the chlorine atom and the 4-position for the fluorine atom); a straight-chain or branched alkyl group having 1 to 6 carbon atoms (the materials enumerated for X and Y are useful, but a straight-chain or branched alkyl group having 1 to 4 carbon atoms is preferable; and the position of this substituent group is not limited to a specific position, but is preferably the 2- or 4-position); a straight-chain or branched alkylcarbonyl group having 2 to 4 carbon atoms (the materials enumerated for X and Y are useful, but a methoxycarbonyl group is preferable; and the position of this substituent group is not limited to a specific position, but is preferably the 4-position); a straight-chain or branched alkylthio group having 1 to 4 carbon atoms (for example, a methylthio group, an ethylthio group, an n-propylthio group, an i-propylthio group, an n-butylthio group, an i-butylthio group, and a t-butylthio group are useful, but a methylthio group is preferable; and the position of this substituent group is not limited to a specific position, but is preferably the 4-position); a nitro group (the position of this substituent group is not limited to a specific position, but is preferably the 4-position); a formyl group (the position of this substituent group is not limited to a specific position, but is preferably the 2-position); and an alkylamino group having 1 to 4 carbon atoms (a dialkylamino group having 1 to 4 carbon atoms is preferable, and a diethylamino group is more preferable; and the position of this substituent group is not limited to a specific position, but is preferably the 3-position).

Examples of the halogen atom are those exemplified for X and Y, but a chlorine atom is preferable.

Examples of the alkyl group having 1 to 6 carbon atoms are those exemplified for X and Y, but a straight-chain or branched alkyl group having 1 to 4 carbon atoms is more preferable. Among them, methyl group and an i-propyl group are most preferable.

A preferable example of the alkenyl group having 2 to 4 carbon atoms is a vinyl group.

W represents a leaving group such as a halogen atom and p-toluenesulfonyloxy.

Since compound (I) has an N,N'-di-substituted piperazine skeleton, an acid-addition salt can be easily formed.

Examples of the acid for forming the acid-addition salt are an inorganic acid (e.g., hydrochloric acid, hydrobromic acid, nitric acid, sulfuric acid, or phosphoric acid), an organic carboxylic acid (formic acid, oxalic acid, fumaric acid, adipic acid, stearic acid, oleic acid, or aconitic acid), an organic sulfonic acid (methanesulfonic acid, benzenesulfonic acid, or p-toluenesulfonic acid), and saccharin.

Compound (I) of the present invention can be synthesized by causing compounds (II) and (III) as starting materials to react with each other in the presence or absence of a solvent. This reaction is preferably performed in the presence of a base to accelerate the reaction.

The solvent is not limited to a specific one as long as it does not directly interfere with the reaction. Examples of the solvent are aromatic, aliphatic, or alicyclic hydrocarbons which are chlorinated or not chlorinated (e.g., benzene, toluene, xylene, methylnaphthaline, petroleum ether, ligroin, hexane, chlorobenzene, dichlorobenzene, methylene chloride, chloroform, dichloromethane, dichloroethane, trichloroethylene, and cyclohexane); ethers (e.g., diethyl ether, tetrahydrofuran, and dioxane); ketones (e.g., acetone and methyl ethyl ketone); amides (e.g., N,N-dimethylformamide and N,N-diethylacetoamide); an organic base (e.g., triethylamine, pyridine, or N,N-dimethylaniline); 1,3-dimethyl-2-imidazolydinone; dimethylsulfoxide; and a mixture of the above solvents.

The content of the solvent is determined such that the content of compound (II) falls within the range of 5 to 80 wt%. Preferably, the solvent is used such that the content of compound (II) falls within the range of 10 to 70 wt%.

The base is not limited to a specific one. Examples of the base are: an organic base (e.g., triethylamine, pyridine, N,N-dimethylaniline, or DBU); an alkali metal alkoxide (e.g., sodium methoxide or sodium ethoxide); and an inorganic base (e.g., hydrogenated sodium, sodium amide, sodium hydroxide, potassium hydroxide, sodium carbonate, sodium bicarbonate, or potassium carbonate). An inorganic base such as potassium carbonate or sodium carbonate is preferable.

The base can be used at a molar ratio of 0.001 to 5 mol per mol of compound (II). A preferable molar ratio is 0.8 to 1.5 mol base per mol of compound (II).

The reaction temperature is not limited to a specific temperature but may range from room temperature to a boiling point of a solvent used. It is however preferable to control the reaction temperature within the range of about 20 °C to 60 °C.

The reaction time varies in accordance with the concentrations of the reactants and the reaction temperature, and generally takes 0.3 to 8 hours.

As for the ratio of the starting materials, compound (III) may be used at a molar ratio of 0.5 to 2 mols, preferably 0.8 to 1.5 mols per mol of compound (II).

Target compound (I) manufactured as described above is subjected after the completion of the reaction to normal post-treatments such as extraction, condensation, and filtration. Compound (I) if required can be appropriately purified by a known means such as recrystallization and various types of chromatography.

Examples of compound (I) (hereinafter referred to as Compounds 1 to 120) are shown in Tables 1.

Table 1

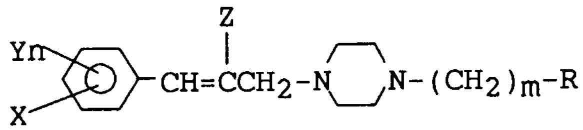

(I)

| Compound | 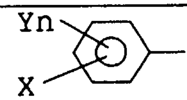 | Z | m | -R | Physical Property |
|---|---|---|---|---|---|
| 1 | 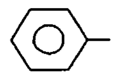 | $CH_3$ | 3 | 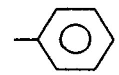 | $n_D^{22}$ 1.5626 |
| 2 | 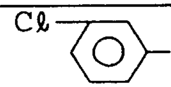 | $CH_3$ | 3 | 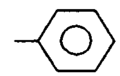 | $n_D^{24}$ 1.5676 |
| 3 | 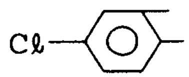 | $CH_3$ | 3 | 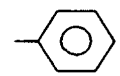 | $n_D^{24}$ 1.5672 |
| 4 | 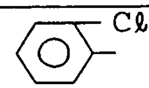 | $CH_3$ | 2 | 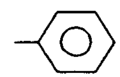 | $n_D^{23}$ 1.5742 |
| 5 | 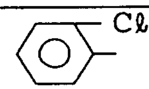 | $CH_3$ | 2 | 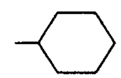 | $n_D^{23}$ 1.5384 |
| 6 | 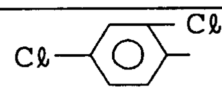 | $CH_3$ | 3 | 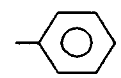 | $n_D^{26}$ 1.5704 |
| 7 | 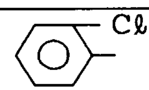 | $CH_3$ | 3 | 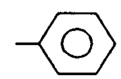 | $n_D^{22}$ 1.5652 |
| 8 | 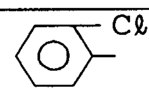 | $CH_3$ | 3 | 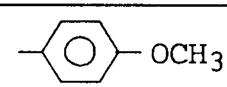 | $n_D^{26}$ 1.5624 |

Table 2

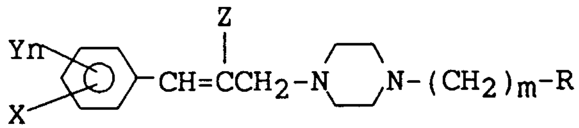 (I)

| Compound | 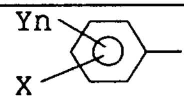 | Z | m | -R | Physical Property |
|---|---|---|---|---|---|
| 9 | 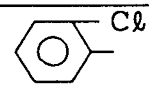 | $CH_3$ | 3 | 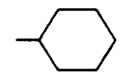 | $n_D^{26}$ 1.5350 |
| 10 | 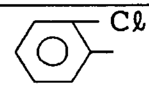 | $i-C_3H_7$ | 3 | 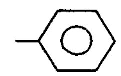 | $n_D^{26}$ 1.5504 |
| 11 | 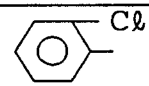 | 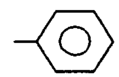 | 2 | 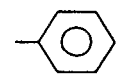 | $n_D^{23}$ 1.6012 |
| 12 | 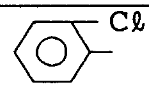 | $CH_3$ | 4 | 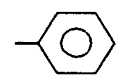 | $n_D^{25}$ 1.5586 |
| 13 | 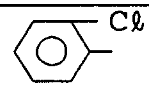 | $CH_3$ | 3 | 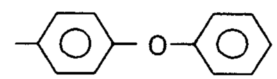 | $n_D^{24}$ 1.5794 |
| 14 | 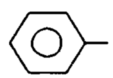 | Br | 2 | 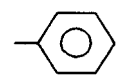 | $n_D^{24}$ 1.5922 |
| 15 | 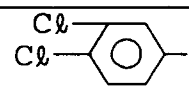 | $CH_3$ | 3 | 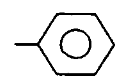 | $n_D^{24}$ 1.5772 |
| 16 | 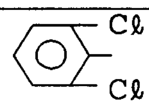 | $CH_3$ | 3 | 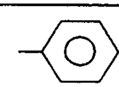 | $n_D^{24}$ 1.5636 |

Table 3

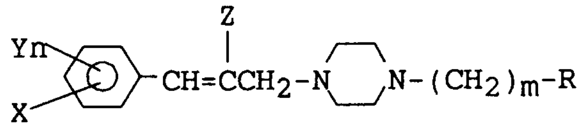 (I)

| Compound | 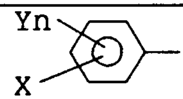 | Z | m | -R | Physical Property |
|---|---|---|---|---|---|
| 17 | 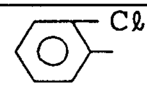 | $C_2H_5$ | 3 | 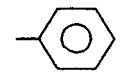 | $n_D^{26}$ 1.5610 |
| 18 | 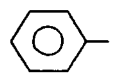 | Br | 3 | 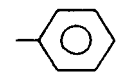 | $n_D^{26}$ 1.5856 |
| 19 | 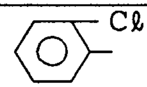 | $n\text{-}C_3H_7$ | 2 | 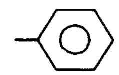 | $n_D^{26}$ 1.5604 |
| 20 | 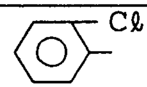 | $C_2H_5$ | 2 | 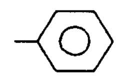 | $n_D^{26}$ 1.5660 |
| 21 | 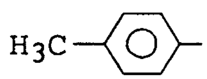 | $CH_3$ | 3 | 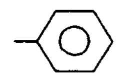 | $n_D^{26}$ 1.5578 |
| 22 | 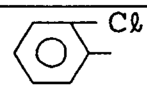 | $i\text{-}C_3H_7$ | 2 | 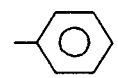 | $n_D^{31}$ 1.5554 |
| 23 | 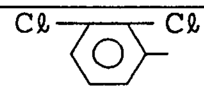 | $CH_3$ | 2 | 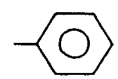 | $n_D^{29}$ 1.5762 |
| 24 | 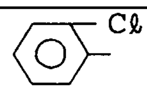 | $n\text{-}C_3H_7$ | 3 | 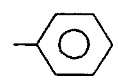 | $n_D^{26}$ 1.5530 |

Table 4

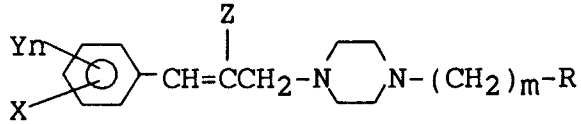

(I)

| Compound | 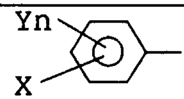 | Z | m | -R | Physical Property |
|---|---|---|---|---|---|
| 25 | 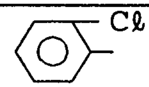 | $CH_3$ | 2 | 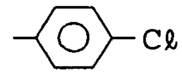 | $n_D^{30}$ 1.5724 |
| 26 | 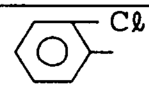 | $CH_3$ | 3 | 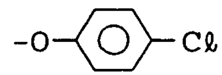 | $n_D^{30}$ 1.5686 |
| 27 | 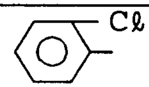 | $CH_3$ | 3 | 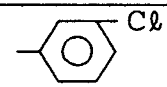 | $n_D^{24}$ 1.5662 |
| 28 | 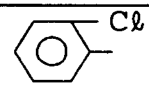 | $CH_3$ | 3 | 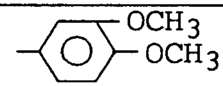 | $n_D^{24}$ 1.5582 |
| 29 | 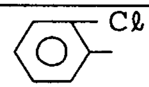 | $CH_3$ | 2 | 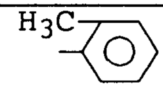 | $n_D^{30}$ 1.5688 |
| 30 | 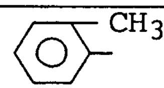 | $CH_3$ | 3 | 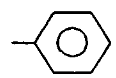 | $n_D^{26}$ 1.5550 |
| 31 | 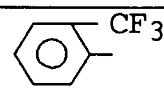 | $CH_3$ | 3 | 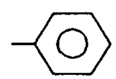 | $n_D^{26}$ 1.5252 |
| 32 | 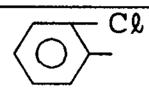 | $CH_3$ | 2 | 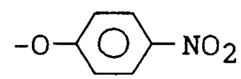 | $n_D^{21}$ 1.5684 |

Table 5

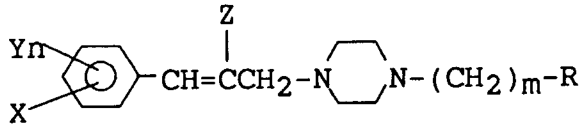

(I)

| Compound | 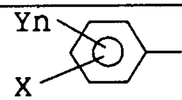 | Z | m | -R | Physical Property |
|---|---|---|---|---|---|
| 33 | 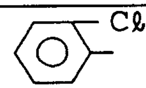 | $CH_3$ | 2 | 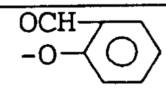 | $n_D^{22}$ 1.5704 |
| 34 | 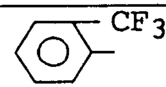 | $CH_3$ | 2 | 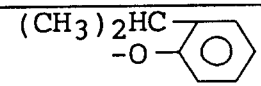 | $n_D^{22}$ 1.5208 |
| 35 | 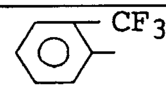 | $CH_3$ | 2 | 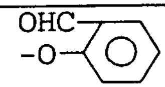 | m.p. 85 to 86°C |
| 36 | 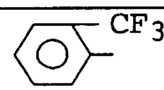 | $CH_3$ | 2 | 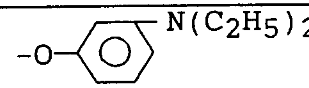 | $n_D^{22}$ 1.5352 |
| 37 | 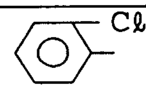 | $CH_3$ | 2 | 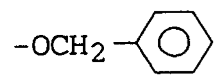 | $n_D^{23}$ 1.5606 |
| 38 | 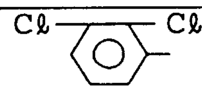 | $CH_3$ | 3 | 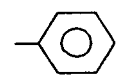 | $n_D^{30}$ 1.5700 |
| 39 | 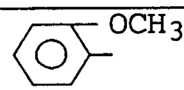 | $CH_3$ | 3 | 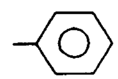 | $n_D^{30}$ 1.5558 |
| 40 | 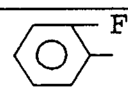 | $CH_3$ | 3 | 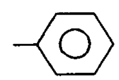 | $n_D^{27}$ 1.5504 |

Table 6

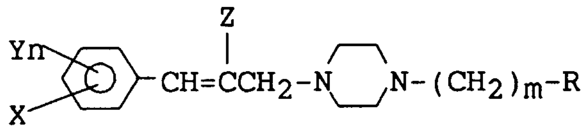 (I)

| Compound | 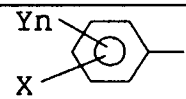 | Z | m | -R | Physical Property |
|---|---|---|---|---|---|
| 41 | 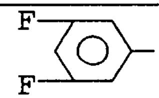 | $CH_3$ | 3 | 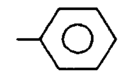 | $n_D^{27}$ 1.5412 |
| 42 | 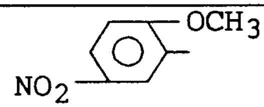 | $CH_3$ | 3 | 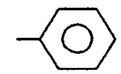 | $n_D^{27}$ 1.5744 |
| 43 | 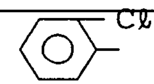 | $n\text{-}C_7H_{15}$ | 2 | 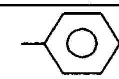 | $n_D^{27}$ 1.5432 |
| 44 | 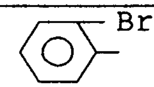 | $CH_3$ | 3 | 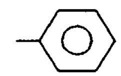 | $n_D^{27}$ 1.5726 |
| 45 | 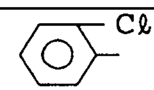 | $CH_3$ | 2 | 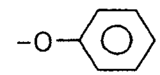 | $n_D^{28}$ 1.5642 |
| 46 | 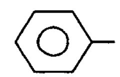 | Cl | 2 | 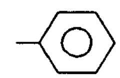 | $n_D^{28}$ 1.5786 |
| 47 | 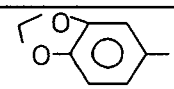 | $CH_3$ | 3 | 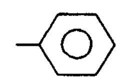 | $n_D^{28}$ 1.5675 |
| 48 | 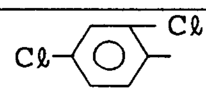 | $n\text{-}C_7H_{15}$ | 2 | 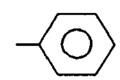 | $n_D^{25}$ 1.5524 |

Table 7

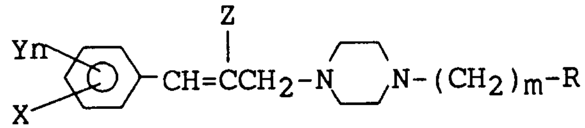

(I)

| Compound | 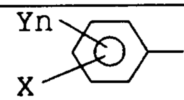 | Z | m | -R | Physical Property |
|---|---|---|---|---|---|
| 49 | 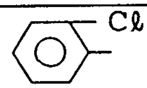 | $CH_3$ | 3 | 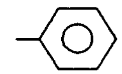 | hydro-chloride |
| 50 | 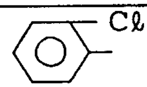 | $CH_3$ | 2 | 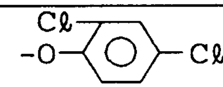 | $n_D^{24}$ 1.5716 |
| 51 | 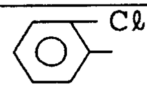 | $CH_3$ | 2 | 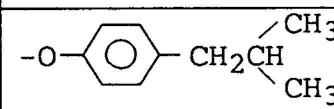 | $n_D^{24}$ 1.5460 |
| 52 | 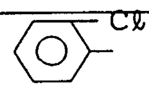 | $CH_3$ | 2 | 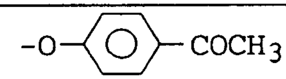 | $n_D^{24}$ 1.5634 |
| 53 | 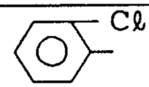 | $CH_3$ | 2 | 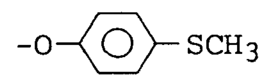 | $n_D^{23}$ 1.5848 |
| 54 | 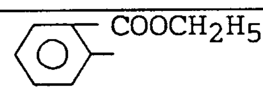 | $CH_3$ | 2 | 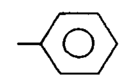 | $n_D^{22}$ 1.5608 |
| 55 | 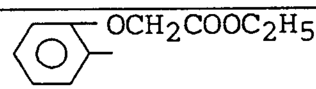 | $CH_3$ | 2 | 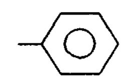 | $n_D^{20}$ 1.5584 |
| 56 | 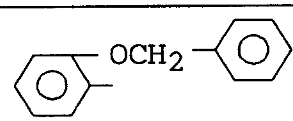 | $CH_3$ | 2 | 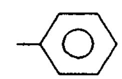 | $n_D^{21}$ 1.5860 |

Table 8

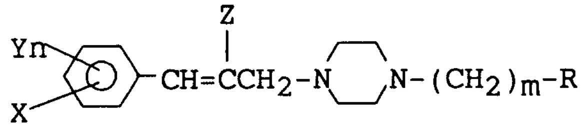 (I)

| Compound | 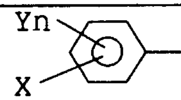 | Z | m | -R | Physical Property |
|---|---|---|---|---|---|
| 57 | 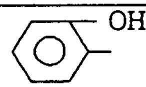 | $CH_3$ | 2 | 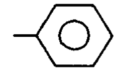 | |
| 58 | 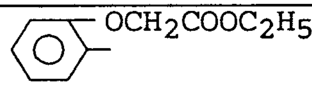 | $CH_3$ | 3 | 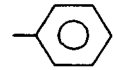 | $n_D^{23}$ 1.5512 |
| 59 | 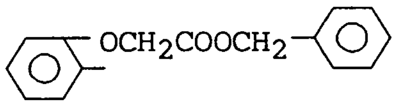 | $CH_3$ | 2 | 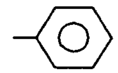 | $n_D^{23}$ 1.5784 |
| 60 | 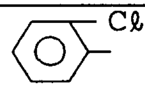 | $CH_3$ | 3 | -Cℓ | $n_D^{20}$ 1.5500 |
| 61 | 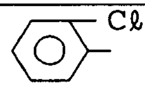 | $CH_3$ | 3 | 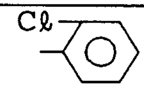 | $n_D^{23}$ 1.5686 |
| 62 | 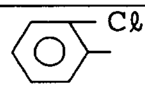 | $CH_3$ | 3 | 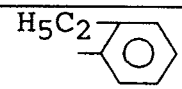 | $n_D^{26}$ 1.5572 |
| 63 | 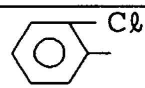 | $CH_3$ | 3 | 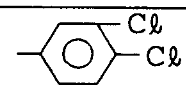 | $n_D^{27}$ 1.5698 |
| 64 | 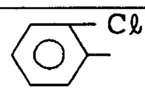 | $CH_3$ | 2 | 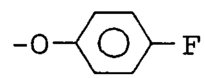 | $n_D^{24}$ 1.5526 |

Table 9

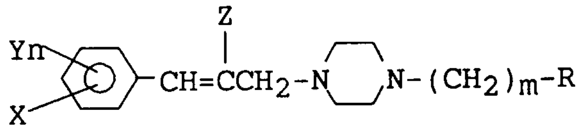 (I)

| Compound | 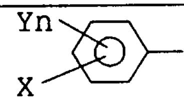 | Z | m | -R | Physical Property |
|---|---|---|---|---|---|
| 65 | 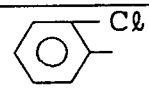 | $CH_3$ | 2 | 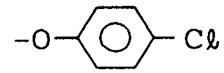 | $n_D^{24}$ 1.5730 |
| 66 | 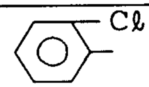 | $CH_3$ | 2 | 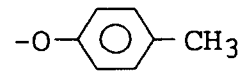 | $n_D^{24}$ 1.5610 |
| 67 | 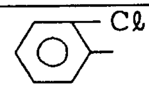 | $CH_3$ | 3 | 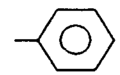 | fumarate; decomposed at 180°C |
| 68 | 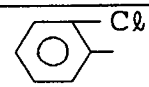 | $CH_3$ | 3 | 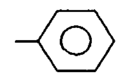 | maleate; decomposed at 160°C |
| 69 | 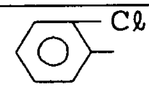 | $CH_3$ | 3 | 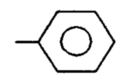 | succinate |
| 70 | 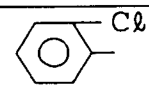 | $CH_3$ | 3 | 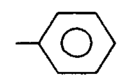 | oxalate |
| 71 | 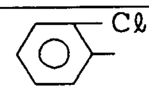 | $CH_3$ | 3 | 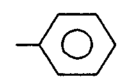 | acetate |
| 72 | 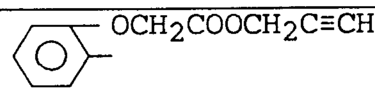 | $CH_3$ | 2 | 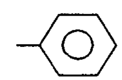 | $n_D^{23}$ 1.5614 |

Table 10

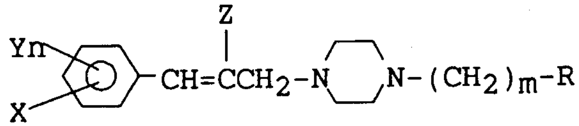 (I)

| Compound | 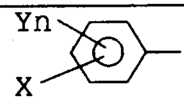 | Z | m | -R | Physical Property |
|---|---|---|---|---|---|
| 73 | 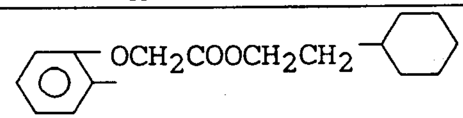 | $CH_3$ | 2 | 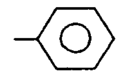 | $n_D^{23}$ 1.5505 |
| 74 | 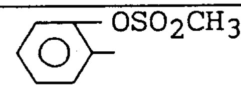 | $CH_3$ | 2 | 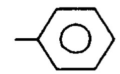 | $n_D^{23}$ 1.5572 |
| 75 | 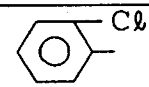 | $CH_3$ | 3 | 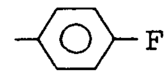 | $n_D^{21}$ 1.5550 |
| 76 | 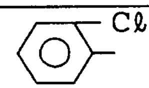 | $CH_3$ | 3 | 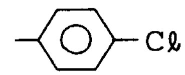 | |
| 77 | 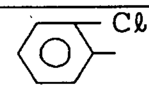 | $CH_3$ | 3 | 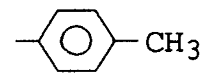 | $n_D^{22}$ 1.5620 |
| 78 | 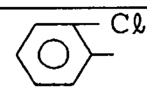 | $CH_3$ | 2 | 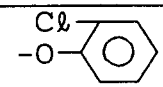 | m.p. 47 to 52°C |
| 79 | 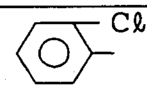 | $CH_3$ | 2 | 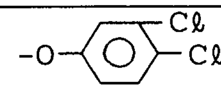 | $n_D^{25}$ 1.5712 |
| 80 | 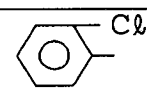 | $CH_3$ | 2 | 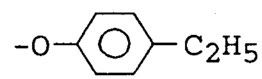 | $n_D^{25}$ 1.5572 |

Table 11

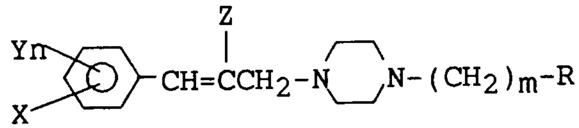 (I)

| Compound | 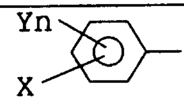 | Z | m | -R | Physical Property |
|---|---|---|---|---|---|
| 81 | 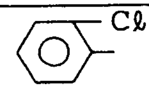 | $CH_3$ | 3 | 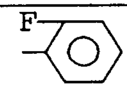 | $n_D^{26}$ 1.5536 |
| 82 | 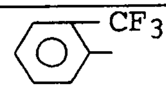 | $CH_3$ | 3 | 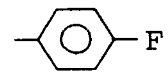 | $n_D^{26}$ 1.5130 |
| 83 | 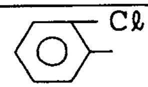 | $CH_3$ | 3 | $-C_3H_7$-i | $n_D^{24}$ 1.5268 |
| 84 | 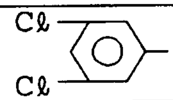 | $CH_3$ | 3 | 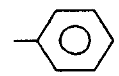 | $n_D^{24}$ 1.5730 |
| 85 | 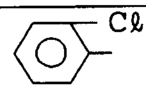 | $CH_3$ | 3 | $-CH=CH_2$ | $n_D^{26}$ 1.5380 |
| 86 | 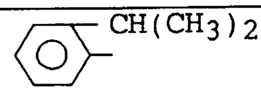 | $CH_3$ | 3 | 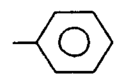 | $n_D^{25}$ 1.5446 |
| 87 | 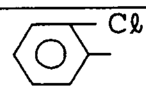 | $CH_3$ | 2 | -S-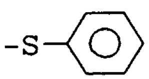 | $n_D^{28}$ 1.5882 |
| 88 | 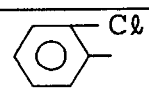 | $CH_3$ | 4 | $-CH=CH_2$ | acetate |

Table 12

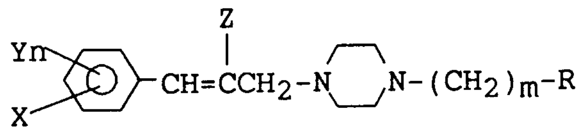 (I)

| Compound | 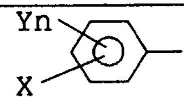 | Z | m | -R | Physical Property |
|---|---|---|---|---|---|
| 89 | 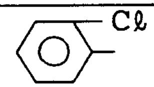 | $CH_3$ | 3 | 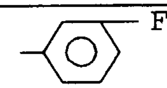 | $n_D^{23}$ 1.5542 |
| 90 | 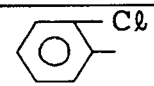 | $CH_3$ | 3 | 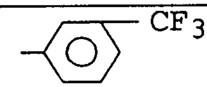 | $n_D^{23}$ 1.5272 |
| 91 | 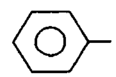 | $CH_3$ | 2 | 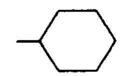 | $n_D^{21}$ 1.5400 |
| 92 | 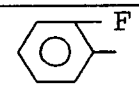 | $CH_3$ | 2 | 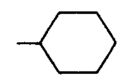 | $n_D^{21}$ 1.5290 |
| 93 | 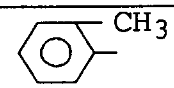 | $CH_3$ | 2 | 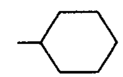 | $n_D^{21}$ 1.5376 |
| 94 | 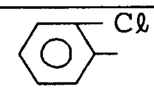 | $CH_3$ | 6 | $-CH_3$ | $n_D^{21}$ 1.5188 |
| 95 | 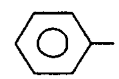 | $CH_3$ | 3 | 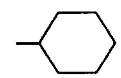 | $n_D^{20}$ 1.5372 |
| 96 | 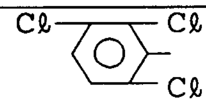 | $CH_3$ | 3 | 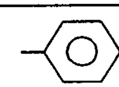 | $n_D^{26}$ 1.5636 |

Table 13

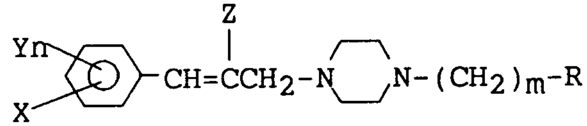

(I)

| Compound | 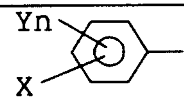 | Z | m | -R | Physical Property |
|---|---|---|---|---|---|
| 97 | 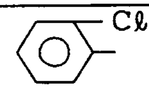 | $CH_3$ | 2 | 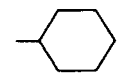 | $n_D^{26}$ 1.5402 |
| 98 | 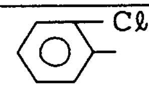 | $CH_3$ | 4 | $-CH=CH_2$ | succinate |
| 99 | 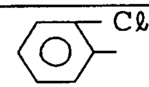 | $CH_3$ | 4 | $-CH_3$ | $n_D^{24}$ 1.5302 |
| 100 | 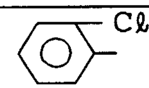 | $CH_3$ | 5 | $-CH_3$ | $n_D^{24}$ 1.5246 |
| 101 | 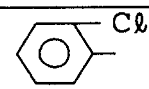 | $CH_3$ | 4 | $-CH=CH_2$ | $n_D^{26}$ 1.5348 |
| 102 | 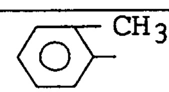 | $CH_3$ | 2 | 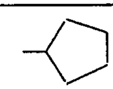 | $n_D^{25}$ 1.5304 |
| 103 | 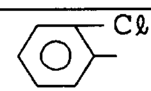 | $CH_3$ | 4 | $-CH=CH_2$ | hydro-chloride |
| 104 | 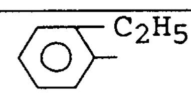 | $CH_3$ | 3 | 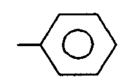 | $n_D^{25}$ 1.5546 |

Table 14

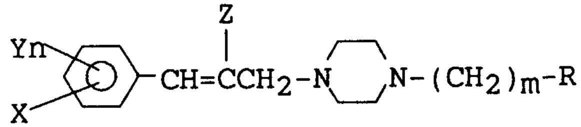 (I)

| Compound | 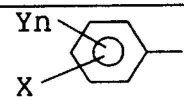 | Z | m | -R | Physical Property |
|---|---|---|---|---|---|
| 105 | 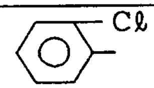 | $CH_3$ | 3 | 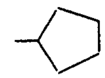 | $n_D^{25}$ 1.5396 |
| 106 | 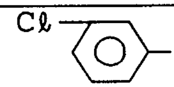 | $CH_3$ | 5 | $-CH_3$ | $n_D^{25}$ 1.5334 |
| 107 | 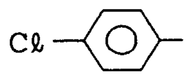 | $CH_3$ | 5 | $-CH_3$ | $n_D^{25}$ 1.5332 |
| 108 | 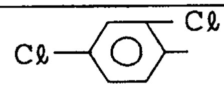 | $CH_3$ | 3 | $-CH=CH_2$ | $n_D^{26}$ 1.5468 |
| 109 | 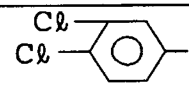 | $CH_3$ | 3 | $-CH=CH_2$ | $n_D^{26}$ 1.5504 |
| 110 | 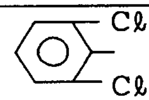 | $CH_3$ | 3 | $-CH=CH_2$ | $n_D^{26}$ 1.5378 |
| 111 | 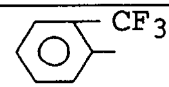 | $CH_3$ | 4 | $-CH_3$ | $n_D^{26}$ 1.4912 |
| 112 | 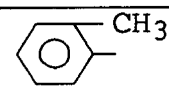 | $CH_3$ | 5 | $-CH_3$ | $n_D^{26}$ 1.5266 |

Table 15

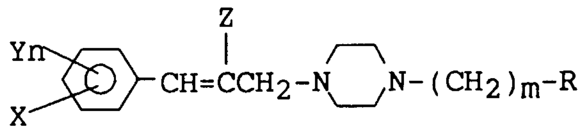

(I)

| Compound | 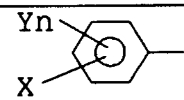 | Z | m | -R | Physical Property |
|---|---|---|---|---|---|
| 113 | 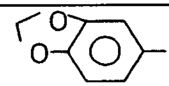 | $CH_3$ | 7 | $-CH_3$ | $n_D^{25}$ 1.5238 |
| 114 | 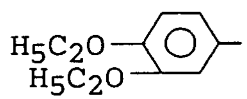 | $CH_3$ | 5 | $-CH_3$ | $n_D^{25}$ 1.5178 |
| 115 | 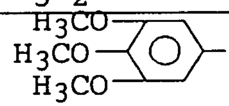 | $CH_3$ | 7 | $-CH_3$ | $n_D^{26}$ 1.5258 |
| 116 | 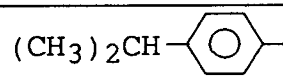 | $CH_3$ | 3 | $-CH_3$ | $n_D^{26}$ 1.5244 |
| 117 | 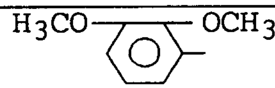 | $CH_3$ | 7 | $-CH_3$ | $n_D^{25}$ 1.5214 |
| 118 | 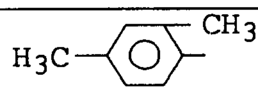 | $CH_3$ | 4 | $-CH_3$ | $n_D^{28}$ 1.5154 |
| 119 | 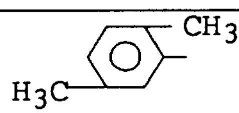 | $CH_3$ | 4 | $-CH_3$ | $n_D^{27}$ 1.5152 |
| 120 | 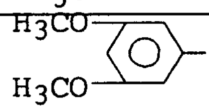 | $CH_3$ | 7 | $-CH_3$ | $n_D^{28}$ 1.5236 |

Compound (II) to be used in the present invention can be easily manufactured by the conventional method of manufacturing tertiary amine (e.g., Published Unexamined European Patent Application No. 266,549). Compound (II) includes several geometrical isomers. Typical examples will be described in Examples 3 and 4.

Compounds (II) synthesized following the same procedures as in Examples 3 and 4 are shown in Table 16.

Table 16

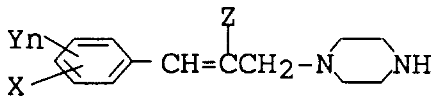

(II)

| 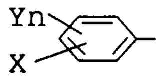 | Z | Physical Property |
|---|---|---|
| 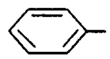 | $CH_3$ | $n_D^{23}$ 1.5592 |
| 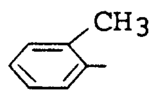 | $CH_3$ | $n_D^{23}$ 1.5498 |
| 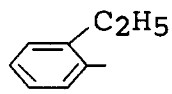 | $CH_3$ | $n_D^{23}$ 1.5410 |
| 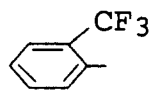 | $CH_3$ | $n_D^{23}$ 1.5090 |
| 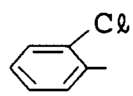 | $CH_3$ | $n_D^{25}$ 1.5630 |

As for compound (II), compounds (hereinafter referred to as Compounds $(II)_1$ to $(II)_{120}$) having substituent groups corresponding to Compounds 1 to 120 shown in Tables 1 to 15 can be used.

Compound (III) to be used in the present invention can be easily manufactured by the conventional haloalkylation or tosylation method.

As for compound (III), compounds (hereinafter referred to as Compounds $(III)_1$ to $(III)_{120}$) having substituent groups corresponding to Compounds 1 to 120 in Tables 1 to 15 and having Br as the leaving group W can be used.

Examples of the agricultural/horticultural pathogenic fungi which can be sterilized by compounds (I) of the present invention are puccinia recondita, erysphea graminis, psudoperonospora cubenis, pyricubaria oryzae, and phytophthora infestans.

The agricultural/horticultural fungicide according to the present invention has a distinguished agricultural/horticultural sterilization effect and contains as an active ingredient at least one cinnamyl piperazine derivative as represented by the formula of compound (I).

Compound (I) can be used singly, but is generally and preferably used as a mixture with a carrier, a surfactant, a dispersant, or an assistant so as to formulate it as a powder, an emulsion, a fine granule, a granule, a water dispersible power, an oily suspension, or an aerosol in accordance with the conventional method.

Examples of the carrier are: a solid carrier such as talc, bentonite, clay, kaolin, siliceous earth, white carbon, vermiculite, slaked lime, quartz sand, ammonium nitrate, or urea; a liquid carrier such as a hydrocarbon (e.g., kerosine or a mineral oil), an aromatic hydrocarbon (e.g., benzene, toluene, or xylene), a chlorinated hydrocarbon (e.g., chloroform or carbon tetrachloride), ethers (e.g., dioxane and tetrahydrofuran), ketones (acetone, cyclohexanone, and isophorone), esters (e.g., ethyl acetate, ethylene glycol acetate, and dibutyl maleate), alcohols (e.g., methanol, n-hexanol, and ethylene glycol), a polar solvent (e.g., dimethylformamide or dimethylsulfoxide), or water; and a gaseous carrier (a mixture can be sprayed in this case) such as air, nitrogen, carbon dioxide gas, or Freon.

Examples of the surfactant or dispersant used to improve adhesion or absorption of the fungicide of the present invention to animals and plants and to improve dispersion, emulsion, and spreading of chemical agents are alcohol sulfates, alkyl sulfonates, lignine sulfonates, and polyoxyethylene glycol ether. In order to improve the physical properties of the resultant compound, carboxymethyl cellulose, polyethylene glycol, acacia gum, or the like can be used as an assistant.

In the manufacture of the fungicide of the present invention, the carrier, the surfactant, the dispersant, and the assistant can be used singly or in a combination depending on the purpose of application.

When the compound (I) of the present invention is used to manufacture a fungicide, the concentration of the active ingredient is, in general, 1 to 50 wt% for an emulsion formula, 0.3 to 25 wt% for a powder formula, 1 to 90 wt% for a water dispersible powder formula, 0.5 to 5 wt% for a granule formula, 0.5 to 5 wt% for an oily suspension formula, and 0.1 to 5 wt% for an aerosol formula.

The above fungicides of the present invention are diluted into an appropriate concentration and are sprayed over the leaves of plants, soil, or the surface of a paddy field, or the fungicides are directly applied to the plants.

The present invention will be described in detail by way of its examples. These examples however should not be understood to limit the scope of the present invention.

Example 1 [Synthesis of Compound (I)]

Synthesis of N-(β-methyl-2-chlorocinnamyl)-N'-(3-phenylpropyl) piperazine (Compound 7)

N-(β-methyl-2-chlorocinnamyl)piperazine (2.5 g, 10 mmol), 1-bromo-3-phenylpropane (2.0 g, 10 mmol), and potassium carbonate (1.5 g, 11 mmol) were dissolved in N,N-dimethylformamide (30 mℓ), and the resultant solution was heated and stirred at 40 °C for 2 hours.

After the reaction, water was added to the reaction solution, and diethyl ether was added thereto to perform extraction. An aqueous 1N hydrochloric acid solution was added to this dimethyl ether layer, and the precipitated salt was filtered. The resultant salt was dissolved in an aqueous 1N sodium hydroxide solution, and dichloromethane was added to perform extraction. The resultant dichloromethane layer was dried with sodium sulfate anhydride, and the solvent was distilled at a reduced pressure.

The resultant residue was separated by a silica gel chromatography (Wakogel C-200; hexane : ethyl acetate = 4 : 1), thereby obtaining 3.0 g of a target product of yellowish oily E/Z isomer mixture.

$n_D^{22}$ 1.5652

CI-MS m/z; 369 ($M^+$ + 1), 333, 263

$^1$H-NMR ($CDCL_3$) $\delta_{ppm}$;

1.75 (s,3H), 1.75 - 1.90 (m, 2H), 2.38 (t, 2H), 2.50 (s, broad, 8H), 2.64 (t, 2H), 3.02 (s, 2H), 6.48 (s, 1H), 7.10 - 7.40 (m, 9H)

Example 2 [Synthesis of Compound (I)]

Synthesis of N-(β-methyl-2-chlorocinnamyl)-N'-(5-hexenyl)pipera zine (Compound 101)

N-(β-methyl-2-chlorocinnamyl)piperazine (2.5 g, 10 mmol), 6-bromo-1-hexene (1.6 g, 10 mmol), and potassium carbonate (1.5 g, 11 mmol) were dissolved in N,N-dimethylformamide (30 mℓ), and the resultant solution was heated and stirred at 40 °C for 2 hours.

After the reaction, water was added to the reaction solution, and diethyl ether was added hereto to perform extraction. The resultant diethyl ether layer was acid-extracted using an aqueous 1N hydrochloric acid solution. This acid extract was converted into an alkaline solution using an aqueous 1N sodium hydroxide solution, and dichloromethane was added thereto, thereby performing extraction. The resultant dichloromethane layer was dried with sodium sulfate anhydride, and the solvent was distilled at a reduced pressure.

The resultant residue was separated by a silica gel chromatography (Wakogel C-200; hexane : ethyl acetate = 4 : 1), thereby obtaining 3.2 g of a target product of transparent oily E/Z isomer mixture.

$n_D^{26}$ 1.5348

EI-MS m/z; 332 ($M^+$), 263, 165, 112 (100%), 70

$^1$H-NMR ($CDCL_3$) $\delta_{ppm}$;

1.36 - 1.70 (m, 4H), 1.78 (s, 3H), 2.00 - 2.15 (m, 2H), 2.36 (t, 2H), 2.52 (s, broad, 8H), 3.05 (s, 2H), 4.86 - 5.10 (m, 2H), 5.70 - 5.90 (m, 1H), 6.48 (s, 1H), 7.10 - 7.40 (m, 4H)

Example 3 [Synthesis of Compound (II)]

Synthesis of N-($\beta$-methyl-2-chlorocinnamyl)piperazine

2-chlorobenzaldehyde (28.1 g, 0.2 mol), propyonaldehyde (11.6 g, 0.2 mol), and potassium hydroxide (1.0 g) were added to ethyl alcohol as a solvent, and the resultant solution was heated and stirred at 50°C for 2 hours. After the reaction, the reaction solution was cooled with ice, and sodium borohydride (2.1 g, 0.055 mol) was added thereto. The resultant mixture was heated to room temperature and stirred. After the reaction, an aqueous 1N-HCl solution was added to the reaction solution, and extra sodium borohydride was removed. The solvent was distilled to obtain 32.9 g of a residue.

This residue was dissolved in ethyl ether, and phosphorous tribromide (17.9 g, 0.066 mol) was dripped therein under ice-cooling. The resultant solution was stirred at room temperature for 2 hours. After the reaction, the reaction solution was treated with a saturated aqueous sodium bicarbonate solution, and the ether layer was dried with sodium sulfate anhydride. The solvent was distilled at a reduced pressure to obtain 44.2 g of a residue.

This residue was dripped together with ethylalcohol in a piperazine (69.7 g, 0.81 mol) ethyl alcohol solution at room temperature. After the resultant solution was stirred for 2 hours, the solvent was distilled, and an aqueous NAOH solution was added to the residue. This solution was subjected to extraction with methylene chloride. The methylene chloride layer was dried with sodium sulfate anhydride, and the solvent was distilled at a reduced pressure. The resultant residue was subjected to a silica gel chromatography to obtain 29.2 g of a target product of light yellowish oily E/Z isomer mixture.

$n_D^{25}$ 1.5630

CI-MS m/z; 251 ($M^+$ + 1), 208, 165

$^1$H-NMR ($CDCl_3$) $\delta_{ppm}$;

1.78 (s, 3H), 2.40 - 2.50 (m, 4H), 2.85 - 2.95 (m, 4H), 3.05 (s, 2H), 6.48 (s, 1H), 7.10 - 7.40 (m, 4H)

Example 4 [Synthesis of Compound (II)]

Synthesis of N-($\beta$-methyl-2-methylcinnamyl)piperazine

2-methylbenzaldehyde (24.0 g, 0.2 mol), propyonaldehyde (11.6 g, 0.2 mol), and potassium hydroxide (1.0 g) were added to ethyl alcohol as a solvent, and the resultant solution was heated and stirred at 50°C for 2 hours. After the reaction, the reaction solution was cooled with ice, and sodium borohydride (2.1 g, 0.055 mol) was added thereto. The resultant mixture was heated to room temperature and stirred. After the reaction, an aqueous 1N-HCl solution was added to the reaction solution to remove sodium borohydride. The solvent was distilled and then the residue was extracted with ethyl acetate/water. The ethyl acetate layer was dried with sodium sulfate anhydride, and ethyl acetate was distilled at a reduced pressure, thereby obtaining 27.5 g of a residue.

This residue was dissolved in ethyl ether, and phosphorous tribromide (17.9 g, 0.066 mol) was dripped therein under ice-cooling. The resultant solution was stirred at room temperature for 2 hours. After the reaction, the reaction solution was treated with a saturated aqueous sodium bicarbonate solution, and the ether layer was dried with sodium sulfate anhydride. The solvent was distilled at a reduced pressure to obtain 34.3 g of a residue.

This residue was dripped together with ethyl alcohol into a piperazine (69.7 g, 0.81 mol) ethyl alcohol solution at room temperature. After the resultant solution was stirred for 2 hours, the solvent was distilled, and an aqueous NAOH solution was added to the residue. This solution was subjected to extraction with methylene chloride. The methylene chloride layer was dried with sodium sulfate anhydride, and the solvent was distilled at a reduced pressure. The resultant residue was subjected to a silica gel chromatography to obtain 31.7 g of a target product of light yellowish oily E/Z isomer mixture.

$n_D$23 1.5498

CI-MS m/z; 231 ($M^+$ + 1), 145, 99

$^1$H-NMR ($CDCl_3$) $\delta_{ppm}$;

1.72 (s, 2H), 1.95 (s, 1H), 2.18 (s, 1H), 2.25 (s, 2H), 2.45 (s, broad, 2H), 2.93 (m, 6H), 3.05 (s, 2H), 6.42 (s, 1H), 7.10 - 7.30 (m, 4H)

Example 5 [Preparation of Fungicide]

(1) Preparation of a granular fungicide

5 parts by weight of Compound 7, 35 parts by weight of bentonite, 57 parts by weight of talc, 1 part by weight of Neopellex Powder (tradename) available from Kao Corp., and 2 parts by weight of sodium ligninesulfonate were uniformly mixed, a small amount of water was added thereto, and the resultant mixture was kneaded, granulated, and dried, thereby obtaining a granular fungicide.

(2) Preparation of a fungicide in the form of water dispersible powder

10 parts by weight of Compound 7, 67.5 parts by weight of kaolin, 20 parts by weight of white carbon, 2 parts by weight of Neopellex Powder (tradename) available from Kao Corp., and 0.5 parts by weight of Demole (tradename) available from Kao Corp. were uniformly mixed and pulverized to obtain a water dispersible powder.

(3) Preparation of a fungicide in the form of emulsion

20 parts by weight of Compound 7, 70 parts by weight of xylenon, and 10 parts by weight of Toxanone (tradename) available from Sanyo Kasei Kogyo were uniformly mixed, and the resultant mixture was dissolved to obtain an emulsion.

(4) Preparation of a fungicide in the form of powder

5 parts by weight of compound 7, 50 parts by weight of talc, and 45 parts by weight of kaolin were uniformly mixed to obtain a powderous fungicide.

Example 6 [Test of Effect]

(1) Control effect test for barley powdery mildew (preventive effect)

Barley seedlings (variety: black barley) were grown in 6-cm diameter plastic flowerpots each having 10 barley seedings. At the 1.5-leaf seedling stage, the water dispersible powder of each of compounds (1) shown in Tables 1 to 15 and prepared following the same procedures as in Example 2 was diluted with water containing a surfactant (0.05%) to 200 ppm or 40 ppm. 20 mℓ of each solution were applied to each flowerpot.

These seedlings were grown in a glass greenhouse for two days, and conidia of erysphea graminis were sampled from leaves infected with the barley mildew. The conidia were uniformly sprinkled and incubated above each seedling.

The infected seedlings were grown for a week in the greenhouse to examine the number of lesions of each first leaf infected with the barley mildew.

The evaluation of the sterilization effect was represented in six levels (0: about 81 to 100% of the lesion area, 1: about 51 to 80% of the lesion area, 2: about 31 to 50% of the legion area, 3: about 11 to 30% of the lesion area, 4: 1 to 10% of the lesion area, and 5: 0% of the lesion area) as compared with the number of lesions of the non-treated leaf.

A comparative fungicide was prepared using the following triforine in place of compound (I):

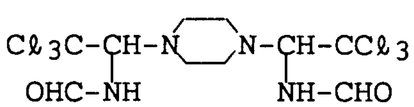

(triforine)

Results are summarized in Table 21.

Table 21

| Control Value for Barley Mildew | | |
|---|---|---|
| Compound | Application Concentration (ppm) | |
| | 200 | 40 |
| 5 | 5 | 5 |
| 7 | 5 | 5 |
| 14 | 5 | 5 |
| 15 | 5 | 5 |
| 16 | 5 | 5 |
| 20 | 5 | 5 |
| 30 | 5 | 5 |
| 31 | 5 | 5 |
| 38 | 5 | 5 |
| 41 | 5 | 5 |
| 49 | 5 | 5 |
| 67 | 5 | 5 |
| 68 | 5 | 5 |
| 69 | 5 | 5 |
| 70 | 5 | 5 |
| 71 | 5 | 5 |
| 77 | 5 | 5 |
| 82 | 5 | 5 |
| 85 | 5 | 5 |
| 89 | 5 | 5 |
| 93 | 5 | 5 |
| 94 | 5 | 5 |
| 97 | 5 | 5 |
| 98 | 5 | 5 |
| 99 | 5 | 5 |
| 100 | 5 | 5 |
| 101 | 5 | 5 |
| 102 | 5 | 5 |
| 103 | 5 | 5 |
| Comparative Fungicide: triforine | 0 | 0 |
| Non-treated | 0 | 0 |

(2) Control effect test for wheat brown rust (preventive effect)

Wheat seedlings (variety: KOBUSHI KOMUGI) were grown in 6-cm diameter plastic flowerpots each having 10 barley seedings. At the 1.5-leaf seedling stage, the water dispersible powder of each of compounds (1) shown in Tables 1 to 15 and prepared following the same procedures as in Example 2 was diluted with water containing a surfactant (0.05%) to 200 ppm or 40 ppm. 20 mℓ of each solution were applied to each flowerpot. These seedlings were grown in a glass greenhouse for two days, and a suspension (2 to 3 × $10^5$/mℓ) of spores of puccinia recondita were uniformly sprinkled for the incnbation thereof.

The infected seedlings were grown for a week in the greenhouse to examine the number of lesions of each first leaf infected to the wheat brown rust.

The evaluation of the sterilization effect was performed using the same comparative fungicide as in (1) of Exampel 6, and indicated according to the the same evaluation method of the six levels as in (1) of this Exampel 6, and results are shown in Table 22.

Table 22

| Control Value for Brown Leaf Rust | | |
|---|---|---|
| Compound | Application Concentration (ppm) | |
| | 200 | 40 |
| 7 | 5 | 4 |
| 24 | 5 | 4 |
| 38 | 5 | 4 |
| 41 | 5 | 5 |
| 49 | 5 | 4 |
| 67 | 5 | 4 |
| 69 | 5 | 4 |
| 71 | 5 | 4 |
| 77 | 5 | 4 |
| 89 | 5 | 5 |
| 90 | 5 | 4 |
| 104 | 5 | 4 |
| Comparative Fungicide: triforine | 0 | 0 |
| Non-treated | 0 | 0 |

(3) Control effect test for cucumber downy mildew

Cucumber seedlings (variation: SAGAMI HANPAKU) were grown in 6-cm diameter plastic flowerpots each having one cucumber seedling. A water dispersible power prepared from each of target compounds (I) of Tables 1 to 15 following the procedures as in Example 2 was diluted with water containing a surfactant (0.05%) to 500 ppm and was applied at a 1.5-leaf seedling stage in an amount of 20 mℓ per flowerpot.

After the application of each fungicide, the cucumber seedlings were grown in a glass greenhouse for two days, and migration sporangia of pseudoperonospora cubenis were sampled from leaves infected with cucumber downy mildew and were uniformly sprayed onto the back surfaces of the leaves for the incubation thereof.

After the incubation, the cucumber seedlings were kept in a dark place at 20°C for two days, and then were grown in the glass greenhouse for 5 days. The number of lesions on each first leaf infected with pseudoperonospora cubensis was counted.

The evaluation of the sterilization effect was performed using the same comparative fungicide as in (1) of Example 6, and indicated according to the same evaluation method of the six levels as in (1) of Example 6, and results are shown in Table 23.

Table 23

| Control Value for Cucumber Downy Mildew | |
|---|---|
| Compound | Concentration Application(ppm) |
| | 500 |
| 4 | 4 |
| 12 | 4 |
| 27 | 5 |
| 28 | 4 |
| 75 | 4 |
| 81 | 4 |
| 82 | 4 |
| 86 | 5 |
| 91 | 5 |
| 92 | 5 |
| 93 | 5 |
| 94 | 5 |
| 98 | 4 |
| 105 | 5 |
| Comparative Fungicide: triforine | 1 |
| Non-treated | 0 |

## Claims

1. A cinnamyl piperazine derivative or an acid-addition salt thereof, the cinnamyl piperazine derivative being represented by formula (I):

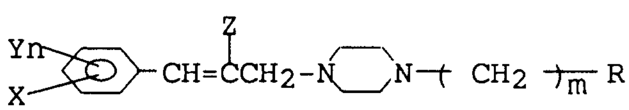

...(I)

(wherein each of X and Y independently represents: a hydrogen atom; a halogen atom; an alkyl group having 1 to 6 carbon atoms; a haloalkyl group having 1 to 4 carbon atoms; an alkoxy group having 1 to 4 carbon atoms, which may have a carbonyl group having an alkoxy group having 1 to 4 carbon atoms, a benzyloxycarbonyl group, a carbonyl group having an alkynyloxy group having 2 to 5 carbon atoms, or a cycloalkylalkyleneoxycarbonyl group having both cycloalkyl having 3 to 10 carbon atoms and alkylene having 1 to 8 carbon atoms; a nitro group; an alkoxycarbonyl group having 2 to 5 carbon atoms; a benzyloxy group; a hydroxyl group; or an alkylsulfonyloxy group having 1 to 4 carbon atoms,

X and Y may be condensed together with carbon atoms to which X and Y are bonded into a benzene ring, thereby forming a 5- or 6-membered ring containing two oxygen atoms,

Z represents an alkyl group having 1 to 10 carbon atoms, a phenyl group, or a halogen atom,

R represents a phenyl group which may have a substituent group, a cycloalkyl group having 3 to 10 carbon atoms, a phenoxy group which may have a substituent group, a benzyloxy group, a halogen atom, an alkyl group having 1 to 6 carbon atoms, an alkenyl group having 2 to 4 carbon atoms, or a phenylthio group,

m represents an integer of 1 to 10, and

n represents 1, 2, or 3).

2. The cinnamyl piperazine derivative or an acid-addition salt thereof according to claim 1, wherein said halogen atom representing X and Y is selected from a chlorine atom, a bromine atom and a fluorine atom.

3. The cinnamyl piperazine derivative or an acid-addition salt thereof according to claim 1, wherein said alkyl group having 1 to 6 carbon atoms representing X and Y is selected from a methyl group, an ethyl group and an i-propyl group.

4. The cinnamyl piperazine derivative or an acid-addition salt thereof according to claim 1, wherein said haloalkyl group having 1 to 4 carbon atoms is trifluoromethyl group.

5. The cinnamyl piperazine derivative or an acid-addition salt thereof according to claim 1, wherein said carbonyl group having alkoxy group having 1 to 4 carbon atoms is a carbonyl group having a methoxy or ethoxy group.

6. The cinnamyl piperazine derivative or an acid-addition salt thereof according to claim 1, wherein said alkynyloxy group is 2-propynyloxy group.

7. The cinnamyl piperazine derivative or an acid-addition salt thereof according to claim 1, wherein said cycloalkyl group having 3 to 10 carbon atoms is cyclohexyl group.

8. The cinnamyl piperazine derivative or an acid-addition salt thereof according to claim 1, wherein said alkylene group having 1 to 8 carbon atoms is ethylene.

9. The cinnamyl piperazine derivative or an acid-addition salt thereof according to claim 1, wherein said alkoxy group having 1 to 4 carbon atoms, which accompanies carbonyl group having alkoxy group is $-OCH_2COOC_2H_5$.

10. The cinnamyl piperazine derivative or an acid-addition salt thereof according to claim 1, wherein said alkoxy group having 1 to 4 carbon atoms, which accompanies benzyloxycarbonyl group is $-OCH_2COOCH_2C_6H_5$.

11. The cinnamyl piperazine derivative or an acid-addition salt thereof according to claim 1, wherein said alkoxy group having 1 to 4 carbon atoms, which accompanies carbonyl group having alkynyloxy group is propalgiloxycarbonylmethoxy.

12. The cinnamyl piperazine derivative or an acid-addition salt thereof according to claim 1, wherein said alkoxy group having 1 to 4 carbon atoms, which accompanies cycloalkylalkyleneoxycarbonyl group is $-OCH_2COOCH_2CH_2C_6H_{11}$.

13. The cinnamyl piperazine derivative or an acid-addition salt thereof according to claim 1, wherein said alkoxycarbonyl group having 2 to 5 carbon atoms representing X and Y is ethoxycarbonyl group.

14. The cinnamyl piperazine derivative or an acid-addition salt thereof according to claim 1, wherein said alkylsulfonyloxy group having 1 to 4 carbon atoms representing X and Y is methylsulfonyloxy group.

15. The cinnamyl piperazine derivative or an acid-addition salt thereof according to claim 1, wherein said Z is selected from alkyl group having 1 to 7 carbon atoms, bromine atom and chlorine atom.

16. The cinnamyl piperazine derivative or an acid-addition salt thereof according to claim 1, wherein said phenyl group representing R is a phenyl group having a substituent group selected from methoxy group, phenoxy group, chlorine atom, fluorine atom, ethyl, methyl and trifluoromethyl group.

17. The cinnamyl piperazine derivative or an acid-addition salt thereof according to claim 1, wherein said cycloalkyl group having 3 to 10 carbon atoms representing R is cyclopentane, or cyclohexane.

18. The cinnamyl piperazine derivative or an acid-addition salt thereof according to claim 1, wherein said phenoxy group representing R is a phenoxy group having a substituent group selected from chlorine atom, fluorine atom, alkyl group having 1 to 6 carbon atoms, methoxycarbonyl group, methylthio group, nitro group, formyl group and diethylamino group.

**19.** The cinnamyl piperazine derivative or an acid-addition salt thereof according to claim 1, wherein said halogen atom representing R is chlorine atom.

**20.** The cinnamyl piperazine derivative or an acid-addition salt thereof according to claim 1, wherein said alkyl group having 1 to 6 carbon atoms representing R is a methyl or i-propyl group.

**21.** The cinnamyl piperazine derivative or an acid-addition salt thereof according to claim 1, wherein said alkenyl group having 2 to 4 carbon atoms representing R is a vinyl group.

**22.** A method of manufacturing a cinnamyl piperazine derivative represented by formula (I) of claim 1, wherein a compound represented by formula (II):

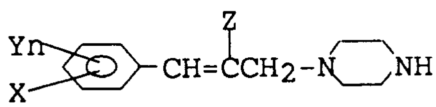

...(II)

(wherein X, Y, Z, and n are the same as defined in claim 1) is reacted with a compound represented by formula (III):

$$W-(CH_2)_m-R \qquad ...(III)$$

(wherein R and m are the same as defined in claim 1, and W represents a leaving group).

**23.** An agricultural/horticultural fungicide containing the cinnamyl piperazine derivative represented by formula (I) or its acid-addition salt as defined in claim 1 as an active ingredient.

**24.** The agricultural/horticultural fungicide according to claim 23, which further contains a carrier, a surfactant, a dispersant, an assistant, or a mixture containing any one of them.